# EUROPEAN PATENT APPLICATION

(11) **EP 4 814 085 A1**
(43) Date of publication of application: **30.09.2026**
(21) Application number: 24894197.3
(22) Date of filing: 21.11.2024
(51) Int. Cl.: C11B 9/00, A61K 8/49, A61Q 13/00, C07D 307/00

(54) **PERFUME, PERFUME COMPOSITION, METHOD FOR PRODUCING PERFUME, AND COMPOUND**

(30) Priority: 24.11.2023 JP 2023199343
(71) Applicant: MITSUBISHI GAS CHEMICAL COMPANY, INC., Chiyoda-ku Tokyo 100-8324 (JP)
(72) Inventor: KITAMURA, Mitsuharu, Tokyo 125-8601 (JP); ASAI, Ryo, Tokyo 125-8601 (JP)
(74) Representative: Müller-Boré & Partner Patentanwälte PartG mbB
(86) International application number: PCT/JP2024/041237
(87) International publication number: WO 2025/110206

(57) **Abstract**

Provided are a fragrance containing a compound useful as a fragrance, a fragrance composition, and a method for producing the fragrance. The fragrance contains a compound represented by Formula (1) or Formula (2). In Formulas (1) and (2), R₁ and R₂ each independently represent a linear, branched, or cyclic alkyl group having from 3 to 12 carbon atoms.

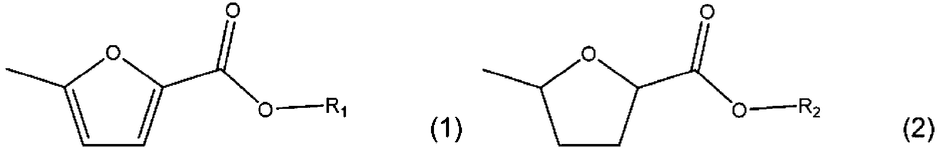

## Description

### Technical Field

The present invention relates to a fragrance, a fragrance composition, a method for producing a fragrance, and a compound.

### Background Art

It is now known that there are a variety of compounds useful as fragrances.

For example, it is known that esters include a compound useful as a fragrance.

Patent Document 1 describes a masking fragrance composition for an unpleasant residual odor during and after hair coloring treatment, or an unpleasant odor generated when wetting the hair after hair coloring treatment.

Patent Document 2 describes an aromatic floor polish containing an emulsion containing an acrylic resin and an aroma component, in which the acrylic resin has a weight average molecular weight of 100000 or more.

Patent Document 3 describes a method for producing an acyloxytetrahydrofuran compound represented by a specific structural formula, in which a compound represented by a specific structural formula is subjected to a catalytic hydrogenation reaction using a palladium catalyst in trifluoroacetic acid or in a specific carboxylic acid solution in which trifluoroacetic acid coexists.

### Citation List

### Patent Documents

Patent Document 1: JP 2020-196678 A
Patent Document 2: JP 2017-008271 A
Patent Document 3: JP S57-158774 A

### Summary of Invention

### Technical Problem

In recent years, consumers' preferences have been diversified, and their demands have also reached the scent of products. In order to cope with such diversity, development of an unknown fragrance component is required.

Issue to be solved by an embodiment of the present invention is to provide a fragrance containing a compound useful as a fragrance, a fragrance composition, and a method for producing a fragrance.

Issue to be solved by another embodiment of the present invention is to provide a novel compound.

### Solution to Problem

The present invention includes the following embodiments:
<1> A fragrance, including a compound represented by Formula (1), and/or a compound represented by Formula (2): 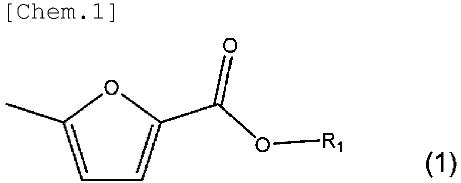 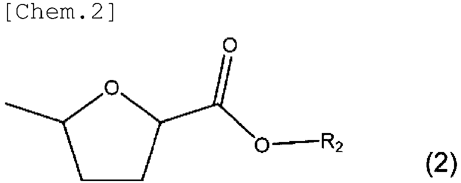 where in Formula (1) or Formula (2), R₁ and R₂ each independently represent a linear, branched, or cyclic alkyl group having from 3 to 12 carbon atoms, wherein the alkyl group optionally contains an unsaturated bond.
<2> The fragrance according to <1>, wherein in Formulas (1) and (2), R₁ and R₂ each independently represent a linear or branched alkyl group having from 3 to 12 carbon atoms.
<3> The fragrance according to <1> or <2>, wherein in Formula (1), R₁ is an n-hexyl group or a 2-ethylhexyl group.
<4> The fragrance according to any one of <1> to <3>, wherein in Formula (2), R₂ is an n-butyl group or an n-hexyl group.
<5> A fragrance composition, containing the fragrance described in any one of <1> to <4>.
<6> The fragrance composition according to <5>, further including at least one fragrance component selected from the group consisting of: hydrocarbons having a terpene skeleton; alcohols; phenols; esters; aldehydes; ketones; acetals; ketals; ethers; nitriles; lactones; hydrocarbons; musks; natural fragrances; natural essential oils; natural extracts; plant extracts; and animal fragrances, with the proviso that compounds corresponding to the compound represented by Formula (1)/or the compound represented by Formula (2) are excluded.
<7> A method for producing a fragrance, the method including:
   a furoate production step of producing methyl-5-methylfuran-2-carboxylate by reacting 5-methylfurfural with methanol; and
   a transesterified furoate production step of producing a fragrance containing a compound represented by Formula (1) set forth below by reacting the methyl-5-methylfuran-2-carboxylate with an alcohol: 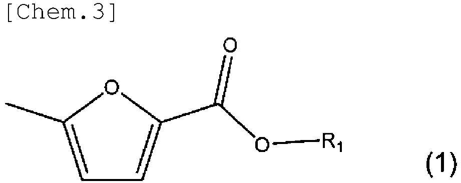
   where in Formula (1), R₁ represents a linear, branched, or cyclic alkyl group having from 3 to 12 carbon atoms.
<8> The method for producing a fragrance according to <7>, wherein in Formula (1), R₁ represents a linear or branched alkyl group having from 3 to 12 carbon atoms.
<9> A method for producing a fragrance, the method including:
   a furoate production step of producing methyl-5-methylfuran-2-carboxylate by reacting 5-methylfurfural with methanol;
   a hydrogenated furoate production step of producing methyl-5-methyltetrahydrofuran-2-carboxylate by reacting the methyl-5-methylfuran-2-carboxylate with H₂; and
   a transesterified hydrogenated furoate production step of producing a fragrance containing a compound represented by Formula (2) set forth below by reacting the methyl-5-methyltetrahydrofuran-2-carboxylate with an alcohol: 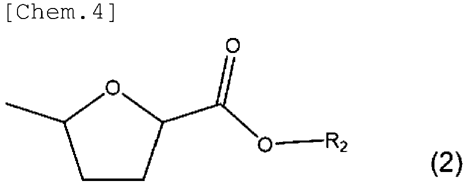
   where in Formula (2), R₂ represents a linear, branched, or cyclic alkyl group having from 3 to 12 carbon atoms.
<10> The method for producing a fragrance according to <9>, wherein in Formula (2), R₂ represents a linear or branched alkyl group having from 3 to 12 carbon atoms.
<11> A compound represented by Formula (2-1) set forth below: 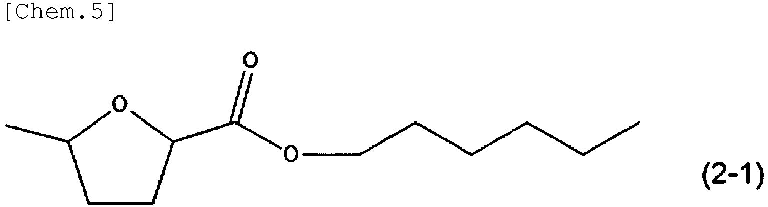
<12> A compound represented by Formula (2-2) set forth below:

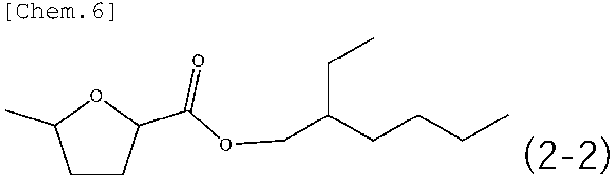

An embodiment of the present invention described above corresponds to embodiments <1> to <10> described above, and another embodiment of the present invention described above corresponds to embodiments <11> to <12> described above.

### Advantageous Effects of Invention

According to an embodiment of the present invention, it is possible to provide a fragrance containing a compound that can be used as a fragrance, a fragrance composition, and a method for producing the fragrance.

In addition, according to another embodiment of the present invention, a novel compound can be provided.

### Description of Embodiments

Embodiments for carrying out the present invention (hereinafter referred to as "the present embodiment") will next be described in detail.

The present embodiment described below is an example for describing the present invention, and the present invention is not limited to the following embodiment. The present invention can be modified as appropriate within the scope of the gist.

In the present embodiment, numerical ranges indicated by "to" mean ranges including the numerical values described before and after the "to" as the minimum and maximum values, respectively.

In the numerical ranges described in the present embodiment in stages, the upper limit or the lower limit described in a certain numerical range may be replaced with the upper limit or the lower limit of another numerical range described in stages. In a numerical range described in the present disclosure, the upper or lower limit value described in a certain numerical range may be replaced by a value presented in the examples.

In the present embodiment, a combination of two or more preferable aspects is a more preferable aspect.

In the present embodiment, in a case where plural kinds of substances corresponding to each component are present, the amount of each component means a total amount of the plural kinds of substances, unless otherwise specified.

The term "step" as used herein includes not only an independent step but also a step that cannot be clearly distinguished from other steps as long as an intended action of the step is achieved. All steps described herein can be performed in any suitable order unless otherwise indicated herein or otherwise clearly contradicted by context.

### <<Fragrance>>

The fragrance of the present embodiment contains a compound represented by Formula (1) or Formula (2).

The compound represented by Formula (1) or Formula (2) can be used as a fragrance component.

It has been found that a compound having a methylfurfural structure or a methyltetrahydrofuran structure, such as the compound represented by Formula (1) or Formula (2), is useful as a fragrance, for example, as a green-like fragrance. Furthermore, it has been found that a compound having a methylfurfural structure or a methyltetrahydrofuran structure and having an ester bond can be used as, for example, a Jasmin-like fragrance in some cases.

In the present embodiment, using the compound represented by Formula (1) or Formula (2) is highly valuable in that, for example, a Jasmin-like fragrance having a green feel can be obtained with a single compound.

In addition, the compound represented by Formula (1) or Formula (2) has high technical value in that such a high-grade fragrance is obtained although the molecular weight is relatively small.

In addition, it is also suitable for enhancing palatability in combination with other fragrances.

### <Compound represented by Formula (1) or Formula (2)>

Hereinafter, the compound represented by Formula (1) or Formula (2) will be described in detail.

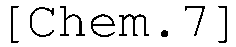

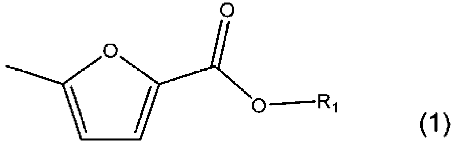

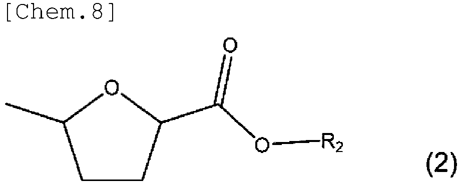

In Formula (1) or Formula (2), R₁ and R₂ each independently represent a linear, branched, or cyclic alkyl group having from 3 to 12 carbon atoms. The alkyl group may contain an unsaturated bond.

Preferably, in Formulas (1) and (2), R₁ and R₂ each independently represent a linear or branched alkyl group having from 3 to 12 carbon atoms.

In Formula (1), the number of carbon atoms in the linear or branched alkyl group is preferably from 4 to 10, more preferably from 5 to 9, and still more preferably from 6 to 8.

In Formula (1), the number of carbon atoms in the cyclic alkyl group is preferably 5 or 6.

Examples of the alkyl group containing an unsaturated bond include propylene and isobutene.

In Formula (1), R₁ is preferably an n-hexyl group or a 2-ethylhexyl group.

In Formula (2), the number of carbon atoms in the linear or branched alkyl group is preferably from 3 to 10 and more preferably from 4 to 8.

In Formula (2), the number of carbon atoms in the cyclic alkyl group is preferably 5 or 6.

In Formula (2), the alkyl group is preferably linear.

In Formula (2), R₂ is preferably an n-butyl group or an n-hexyl group.

The molecular weight of the compound represented by Formula (1) or Formula (2) is preferably 140 or more, more preferably 154 or more, and is preferably 280 or less, more preferably 266 or less. When the molecular weight is equal to or higher than the above-described lower limit or more, the lasting scent tends to further improve. When the molecular weight is equal to or lower than the above-described upper limit or less, it is easy to volatilize, and it is possible to easily feel the aroma. In addition, a greasy feel tends to feel less.

Hereinafter, specific examples of the compound represented by Formula (1) or Formula (2) are presented. Note that the compound used in the fragrance of the present embodiment is not limited to the following compounds:

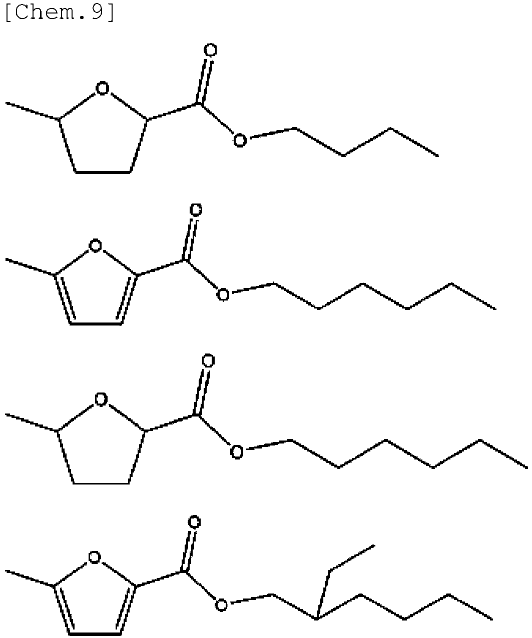

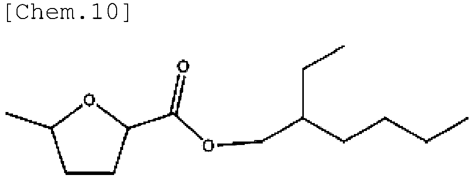

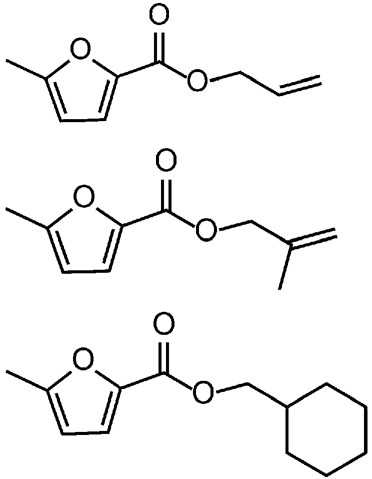

The content of the compound represented by Formula (1) or Formula (2) may be 80 mass% or more, 90 mass% or more, or 100 mass% based on a total mass of the fragrance.

### <<Fragrance composition>>

The fragrance composition of the present embodiment contains the fragrance of the present embodiment.

Aspects of the fragrance composition of the present embodiment are not particularly limited, as long as the fragrance composition contains the fragrance of the present embodiment.

For example, the fragrance composition of the present embodiment may contain components other than the fragrance.

As the other components, for example, other fragrance components (for example, a perfuming component) other than the fragrance of the present embodiment may be contained.

Examples of the other fragrance component include hydrocarbons having a terpene skeleton and the like, alcohols, phenols, esters, aldehydes, ketones, acetals, ketals, ethers, nitriles, lactones, hydrocarbons, musks, natural fragrances, natural essential oils or natural extracts, plant extracts, and animal fragrances.

In addition, for example, other fragrance components described in Comprehensive Overview of Flavor and/or Fragrance Chemicals 1, 2, and 3 (Osamu OKUDA, published by Hirokawa-Shoten Ltd.), Synthetic Fragrances (Motoichi INDO, Chemicals and Product Knowledge), "Japan Patent Office, Compendium of Well-known and Commonly Used Technologies, Fragrances, Chapter III, Cosmetic Fragrances, PP. 26-103, published June 15, 2001", etc. may be used.

Specific examples of the other fragrance component include hydrocarbons such as limonene, α-pinene, β-pinene, terpinene, cedrene, longifolene, and valencene; alcohols such as linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-methyl-2-(2-methylpropyl)tetrahydro-2H-pyran-4-ol, 2-methyl-4-(2,2,3-trimethyl-3-cyclopenten-1-yl)-2-butene-1-ol, 2-ethyl-4-(2,2,3-trimethyl-3-cyclopenten-1-yl)-2-buten-1-ol,isocamphylcyclohexanol, and 3,7-dimethyl-7-methoxyoctane-2-ol; phenols such as eugenol, thymol, vanillin, and vanitrope; esters such as linalyl formate, citronellyl formate, geranyl formate, n-hexyl acetate, cis-3-hexenyl acetate, linalyl acetate, citronellyl acetate, geranyl acetate, neryl acetate, terpinyl acetate, nopyl acetate, bornyl acetate, isobronyl acetate, o-t-butylcyclohexyl acetate (also referred to as Floramat (trade name)), p-t-butylcyclohexyl acetate, tricyclodecenyl acetate, benzyl acetate, styralyl acetate, cinnamyl acetate, dimethylbenzylcarbinyl acetate (also referred to as dimethyl benzyl carbinyl acetate), dimethylbenzylcarbinyl butyrate (also referred to as dimethyl benzyl carbinyl butyrate), 3-pentyltetrahydropyran-4-yl acetate, citronellyl propionate, tricyclodecenyl propionate, allylcyclohexyl propionate, ethyl-2-cyclohexyl propionate, benzyl propionate, benzylbutyrate (also referred to as benzyl butyrate), citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate; aldehydes such as n-octanal, n-decanal, n-dodecanal, 2-methylundecanal, 10-undecenal, citronellal, citral, hydroxycitronellal, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde; ketones such as methylheptenone, 4-methylene-3,5,6,6-tetramethyl-2-heptanone, amylcyclopentanone, 3-methyl-2-(cis-2-pentene-1-yl)-2-cyclopentene-1-on, methylcyclopentenolone, rose ketones, γ-methylionone, α-ionone, carbone, menthone, camphor, nootkatone, benzylacetone, anisylacetone, methyl-β-naphthylketone, 2,5-dimethyl-4-hydroxy-3(2H)-furanone, maltol, ethyl maltol, 7-acetyl-1,2,3,4,5,6,7,8-octahydro-1,1,6,7-tetramethyl naphthalene, muscone, civetone, cyclopentadecanone, and cyclohexedecanone; acetals and ketals such as acetoaldehyde ethylphenylpropyl acetal, citraldiethyl acetal, phenylacetoaldehyde glycerin acetal, and ethylacetoacetate ethyleneglycol ketals; ethers such as anethole, β-naphthylmethyl ether, β-naphthylethyl ether, limonene oxide, rose oxide, 1,8-cineol, and racemic or photoactive dodecahydro-3a,6,6,9a-tetramethylnaphtho[2, 1-b]furane; nitriles such as citronellyl nitrile; lactones such as γ-nonalactone, γ-undecalactone, σ-decalactone, γ-jasmolactone, coumarin, cyclopentadecanolide, cyclohexadecanolide, ambrettolide, ethylene brassylate, and 11-oxahexadecanolide; and natural essential oils and natural extracts such as orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, vetiver, patchouli, and labdanum.

One type of these can be used singly or two or more types thereof can be used in combination.

The fragrance composition of the present embodiment may contain various additives (those that do not function as fragrance components) as necessary, in addition to the other fragrance components described above.

The various additives may be, for example, additives used in cosmetics, health and hygiene materials, daily necessities, miscellaneous goods, fibers, textile products, clothing, foods, quasi-drugs, pharmaceuticals, and the like.

Specific examples of the various additives include solvents and/or dispersion media such as dipropylene glycol, diethyl phthalate, ethylene glycol, propylene glycol, methyl myristate, and triethyl citrate, perticulates (powders), liquid fats and oils, solid fats and oils, waxes, oil-soluble components, silicones, hydrocarbons, higher fatty acids, higher alcohols, lower alcohols, polyhydric alcohols, esters, glycols, alcohol ethers, saccharides, amino acids, organic amines, polymer emulsions, pH adjusting agents, skin nutrients, vitamins, anionic surfactants such as polyoxyethylene lauryl sulfate ether, cationic surfactants, amphoteric surfactants, nonionic surfactants, ultraviolet absorbers, oil gelling agents, humectants, aqueous components, propellants, antioxidants, antioxidation aids, beauty ingredients, preservatives, watersoluble polymers, water, film-forming agents, fragrance fixatives, thickeners, antifoaming agents, disinfectants, deodorants, dyes, pigments, pearl agents, chelating agents, and gelling agents. One type of these can be used singly or two or more types thereof can be used in combination.

The fragrance composition of the present embodiment can be used in any form depending on the various additives to be blended.

The fragrance composition of the present embodiment can be used, for example, in the form of liquid, gel, semi-solid, jelly, solid, powder, mist, aerosol, emulsion, or suspension. Further, the fragrance composition of the present embodiment can also be used in a form in which a base material such as organic or inorganic fibers such as yarns, woven or knitted fabrics, woven fabrics, nonwoven fabrics, and paper, a resin, a clothing material, and clothing is sprayed, applied, adsorbed, mixed, dispersed, emulsified, kneaded, carried, permeated, or impregnated.

Furthermore, the fragrance composition of the present embodiment can also be imparted using a microcapsule and the like. In the fragrance and the fragrance composition of the present embodiment, the scent can be spread or diffused using a diffuser.

The content of the compound represented by Formula (1) or Formula (2) in the fragrance composition of the present embodiment can be appropriately set according to the type and strength of aroma to be aimed, the type and amount of other fragrance components to be used in combination, desired aroma persistence, use form, and the like.

The content of the compound represented by Formula (1) or Formula (2) in the fragrance composition of the present embodiment is preferably 0.01 mass% to 90 mass%, and more preferably 0.1 mass% to 50 mass% based on the total amount of the fragrance composition.

### [Application]

The application of the fragrance composition of the present embodiment is not particularly limited, and suitable examples thereof include cosmetics, food additives, and cleaning.

The fragrance of the present embodiment can be widely used, for example, as an aroma component (perfuming component) of various products such as cosmetics, health and hygiene materials, daily necessities, miscellaneous goods, fibers, textile products, clothing products, foods, quasi-drugs, and pharmaceuticals alone or as a blended fragrance material. The fragrance can also be used to improve the aroma of a product to be formulated.

Specific examples of the various products include fragrance products, basic cosmetics, finishing cosmetics, hair cosmetics, hair care cosmetics, skin cosmetics, suntan cosmetics, medicinal cosmetics, soaps, body cleaners, bath agents, detergents, softeners, bleaching agents, disinfectant detergents, deodorant detergents, furniture cares, various cleaning agents, glass cleaners, furniture cleaners, floor cleaners, disinfectants, insecticides, bleaching agents, aerosols, deodorants, aromatic agents, deodorizing air fresher, repellents, and other miscellaneous goods.

More specific examples include a perfume, a parfum, an eau de parfum, an eau de toilette, colognes, a fragrance powder, a kneaded perfume, a shampoo, hair conditioners, hair rinses, a rinse-in-shampoo, a hair tonic, hair creams, brilliantine, a set lotion, a hair stick, a hair solid, a hair oil, a hair mousse, a hair gel, a hair pomade, a hair liquid, a hair spray, a hair color, a hair pack, a hair growing agent, a hair dyeing agent, a skin lotion, emulsion, a body lotion, a body powder, a body soap, a hand soap, a hand cream, a body cream, an aromatic oil, a liquid cosmetic, a cream, emulsion, a pack, a foundation, a face powder, a lipstick, a cleansing foam, a face wash cream, a make-up remover, a pack, a vanishing cream, a cleansing cream, a cold cream, a massage cream, oil-blotting paper, a foundation, an eye shadow, an eyeliner, a mascara, a lipstick, a makeup base, a loose face powder, a pressed face powder, a talcum powder, a lip balm, a cheek rouge, an eyebrow color, an eye pack, a nail enamel, an enamel remover, a toilet soap, a bath soap, a perfume soap, a transparent soap, a synthetic soap, a liquid soap, a bath salt, a bath tablet, a bath liquid, a foam bath, a bath oil, a bath capsule), a milk bath, a bath jelly, a bath cube, an antiperspirant, a lip balm, a shaving foam, an after-shaving lotion, an after-shaving gel, a hair growth tonic, a permanent wave agent, a medicated soap, a medicated shampoo, a medicated skin cosmetic, a dish soap, a kitchen detergent, a dish detergent, a laundry detergent, a heavy duty detergent, a light duty detergent, a liquid detergent, a compact detergent, a powder soap, softeners, a furniture care, disinfecting detergents, anti-odor detergents, a drain-pipe cleaning agent, an oxidized bleaching agent, a reduced bleaching agent, an optical bleaching agent, aerosols, deodorizers in solid/gel/liquid form, air fresheners in solid/gel/liquid form, deodorizing air freshener in solid/gel/liquid form, a cleanser, a glass cleaner, a furniture cleaner, a leather cleaner, a floor cleaner, a house cleaner, a detergent for fiber, a leather detergent, a toilet cleaner, a bath cleaner, a glass cleaner, a mold remover, a furniture care, a glass cleaner, a furniture cleaner, a floor cleaner, a disinfectant, an insecticide, a toothpaste, a mouthwash, a bath additive, antiperspirant products, a sunscreen, a perming liquid, a hair removal agent, an ointment, a poultice, an ointment agent, a patch, a hair growth agent, a gargle, toilet paper, tissue paper, scented paper, a room fragrance, an aromatic candle, and an aromatic oil.

### <<Method for producing fragrance>>

A method for producing a fragrance of the present embodiment includes production method A for producing a fragrance containing a compound represented by Formula (1), and production method B for producing a fragrance containing a compound represented by Formula (2).

### <Production method A>

Production method A includes a furoate production step of producing methyl-5-methylfuran-2-carboxylate by reacting 5-methylfurfural with methanol, and a transesterified furoate production step of producing a fragrance containing a compound represented by Formula (1) set forth below by reacting the methyl-5-methylfuran-2-carboxylate with an alcohol:

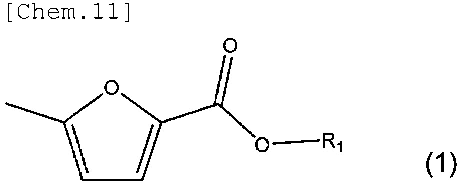

In Formula (1), R₁ represents a linear, branched, or cyclic alkyl group having from 3 to 12 carbon atoms.

Preferably, in Formula (1), R₁ represents a linear or branched alkyl group having from 3 to 12 carbon atoms.

### (Furoate production step)

The furoate production step is a step of producing methyl-5-methylfuran-2-carboxylate by reacting 5-methylfurfural with methanol.

The content of methanol relative to 5-methylfurfural is preferably from 1.0 mass% to 10.0 mass%, more preferably from 2.0 mass% to 7.0 mass%, and still more preferably from 2.5 mass% to 4.5 mass%.

In the furoate production step, methanol may be a solvent.

In the furoate production step, for example, 5-methylfurfural may be reacted with methanol using a catalyst (for example, NaCN) and an oxidizing agent (for example, MnO₂) to produce methyl-5-methylfuran-2-carboxylate. Specifically, 5-methylfurfural may be reacted with the catalyst and then oxidized with an oxidizing agent.

In the reaction of 5-methylfurfural with the catalyst, the reaction time is not particularly limited, and is preferably from 3 minutes to 30 minutes, more preferably from 5 minutes to 20 minutes, and still more preferably from 7 minutes to 15 minutes.

In the reaction of 5-methylfurfural with the catalyst, the reaction temperature is not particularly limited, and is preferably between 15°C and 60°C, and more preferably between 20°C and 40°C.

In the reaction of 5-methylfurfural with the catalyst, the stirring speed is not particularly limited, and is preferably from 100 rpm to 500 rpm, and more preferably from 200 rpm to 400 rpm.

In the oxidation reaction with the oxidizing agent, the reaction time is not particularly limited, and is preferably from 1 hour to 10 hours, more preferably from 2 hours to 8 hours, and still more preferably from 3 hours to 6 hours.

In the oxidation reaction with the oxidizing agent, the reaction temperature is not particularly limited, and is preferably between 1°C and 60°C, and more preferably between 2°C and 50°C.

In the oxidation reaction with the oxidizing agent, the stirring speed is not particularly limited, and is preferably from 100 rpm to 500 rpm, and more preferably from 200 rpm to 400 rpm.

The furoate production step may be performed, for example, by the following method.

As indicated in the following reaction formula, 5-methylfurfural, NaCN as a catalyst, and methanol as a solvent are mixed, and stirred at 300 rpm (revolutions per minute) at 25°C for 10 minutes. After confirming that NaCN is dissolved, the reaction liquid is cooled, and Na₂CO₃ and MnO₂ are charged little by little while maintaining the temperature between 2°C and 10°C. Thereafter, the temperature of the reaction liquid was raised to 40°C to perform the reaction.

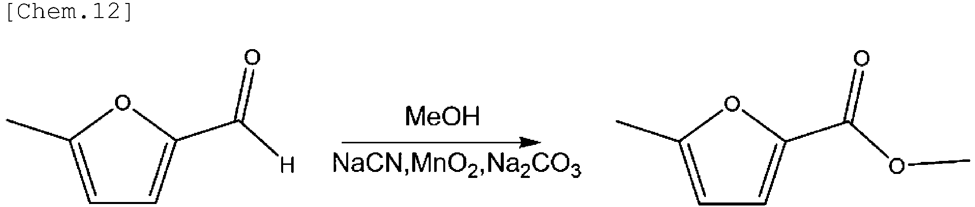

### (Transesterified furoate production step)

The transesterified furoate production step is a step of producing a fragrance containing the compound represented by Formula (1) set forth below by reacting the methyl-5-methylfuran-2-carboxylate with an alcohol.

Examples of the alcohol include linear or branched alcohols having from 3 to 12 carbon atoms.

The number of carbon atoms in the alcohol is preferably from 4 to 10, more preferably from 5 to 9, and still more preferably from 6 to 8.

The alcohol is preferably n-hexanol or 2-ethylhexanol.

The content of the alcohol relative to methyl-5-methylfuran-2-carboxylate is preferably from 1.0 molar equivalent to 5.0 molar equivalent, more preferably from 1.5 molar equivalent to 4.0 molar equivalent, and still more preferably from 2.0 molar equivalent to 3.5 molar equivalent.

In the transesterified furoate production step, a catalyst may be used.

Examples of the catalyst include inorganic acids such as sulfuric acid, phosphoric acid, and hydrochloric acid, and organic sulfonic acids such as p-toluenesulfonic acid, benzenesulfonic acid, and methanesulfonic acid. Among them, the catalyst is preferably p-toluenesulfonic acid.

The content of the catalyst relative to methyl-5-methylfuran-2-carboxylate is preferably from 1 mass% to 10 mass%, more preferably from 2 mass% to 7 mass%, and still more preferably from 3 mass% to 6 mass%.

In the transesterified furoate production step, the reaction time is not particularly limited, and is preferably from 1 hour to 10 hours, more preferably from 2 hours to 8 hours, and still more preferably from 3 hours to 6 hours.

In the transesterified furoate production step, the reaction temperature is not particularly limited, and is preferably between 60°C and 250°C, and more preferably between 100°C and 200°C.

In the transesterified furoate production step, the stirring speed is not particularly limited, and is preferably from 300 rpm to 800 rpm, and more preferably from 400 rpm to 700 rpm.

The transesterified furoate production step may be performed, for example, by the following method.

As indicated in the following reaction formula, methyl-5-methylfuran-2-carboxylate prepared above, an alcohol, and p-toluenesulfonic acid as a catalyst are mixed in a two-necked flask, and the mixture is reacted at 150°C for 4 hours at 500 rpm. Methanol produced as a by-product in the reaction may be removed to the outside of the system during the reaction.

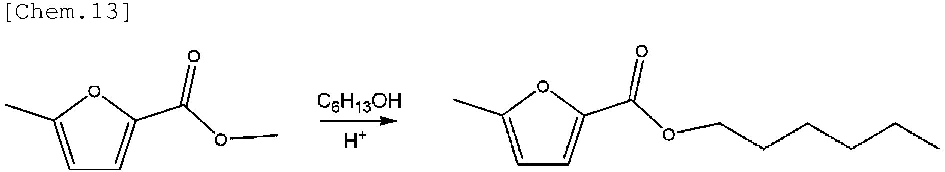

### <Production method B>

Production method B includes a furoate production step of producing methyl-5-methylfuran-2-carboxylate by reacting 5-methylfurfural with methanol;
a hydrogenated furoate production step of producing methyl-5-methyltetrahydrofuran-2-carboxylate by reacting the methyl-5-methylfuran-2-carboxylate with H₂; and
a transesterified hydrogenated furoate production step of producing a fragrance containing a compound represented by Formula (2) set forth below by reacting the methyl-5-methyltetrahydrofuran-2-carboxylate with an alcohol.

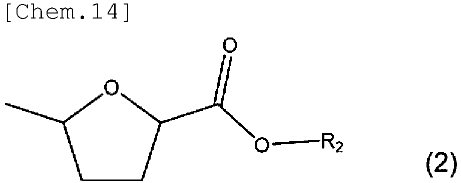

In Formula (2), R₂ represents a linear, branched, or cyclic alkyl group having from 3 to 12 carbon atoms.

Preferably, in Formula (2), R₂ represents a linear or branched alkyl group having from 3 to 12 carbon atoms.

### (Furoate production step)

The furoate production step is a step of producing methyl-5-methylfuran-2-carboxylate by reacting 5-methylfurfural with methanol.

The details of the specific aspect, preferred aspect, and the like of the furoate production step in production method B are the same as the details of the specific aspect, preferred aspect, and the like of the furoate production step in production method A described above.

### (Hydrogenated furoate production step)

The hydrogenated furoate production step is a step of producing methyl-5-methyltetrahydrofuran-2-carboxylate by reacting the methyl-5-methylfuran-2-carboxylate with H₂.

In the hydrogenated furoate production step, a catalyst may be used.

Examples of the catalyst include a hydrogenation catalyst. Examples of the hydrogenation catalyst include metal powders, complexes, and oxide powders of Group 8 metals such as palladium, platinum, ruthenium, rhodium, iridium, cobalt, and nickel, and supported catalysts in which the Group 8 metal is supported on a carrier such as activated carbon, silica gel, or alumina. In the supported catalyst, the content of the Group 8 metal based on the total mass of the carrier is not particularly limited, and may be from 0.5 mass% to 20 mass%.

Among them, the hydrogenation catalyst is preferably activated carbon-supported rhodium, activated carbon-supported ruthenium, alumina-supported ruthenium, alumina-supported rhodium, or Raney nickel.

The content of the catalyst relative to methyl-5-methylfuran-2-carboxylate is preferably from 1 mass% to 15 mass%, more preferably from 3 mass% to 10 mass%, and still more preferably from 4 mass% to 8 mass%.

In the hydrogenated furoate production step, the reaction pressure is preferably from 1.0 MPa to 15.0 MPa, more preferably from 2.0 MPa to 10.0 MPa, and still more preferably from 3.0 MPa to 7.0 MPa.

In the hydrogenated furoate production step, the reaction time is not particularly limited, and is preferably from 1 hour to 15 hours, more preferably from 3 hours to 12 hours, and still more preferably from 5 hours to 10 hours.

In the hydrogenated furoate production step, the reaction temperature is not particularly limited, and is preferably between 50°C and 200°C, and more preferably between 70°C and 120°C.

The hydrogenated furoate production step may be performed, for example, by the following method.

As indicated in the following reaction formula, methyl-5-methylfuran-2-carboxylate prepared above and a 5% Rh/C catalyst are charged into an autoclave, and after replacement with nitrogen gas is performed three times, an airtightness test is performed at 6 MPa. After the pressure drop, the inside of the system is pressurized with H₂ gas, and the reaction is performed at 90°C and 4.5 MPa for 7.5 hours to produce the desired methyl-5-methyltetrahydrofuran-2-carboxylate.

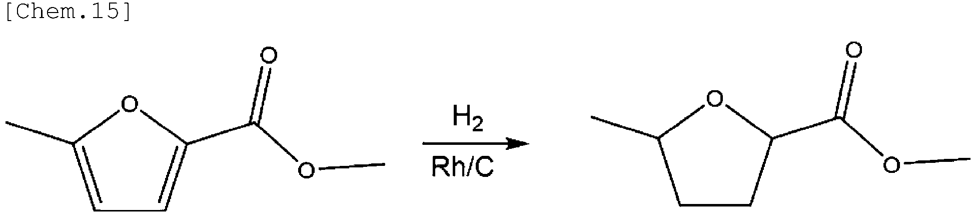

### <<Compound>>

The compound of the present embodiment is a compound represented by Formula (2-1) set forth below:

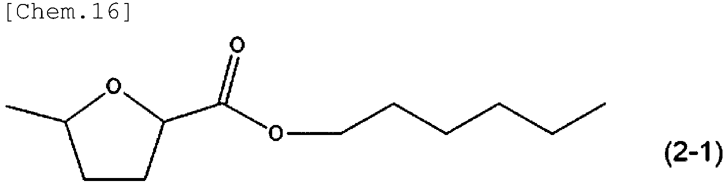

Another example of the compound of the present embodiment is a compound represented by Formula (2-2) set forth below:

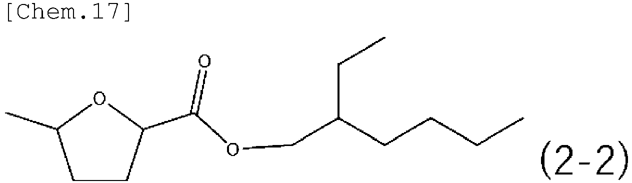

The compound of the present embodiment is a novel compound, and can be used as a fragrance component.

The fragrance composition containing the compound of the present embodiment exhibits a smooth floral feel and diffusibility with a natural feel.

### Examples

Hereinafter, the present embodiment will be described in more detail with reference to specific examples and comparative examples, but the present invention is not limited by the following examples and comparative examples at all.

The measurement method in this example is described.

### <Gas chromatography analysis conditions>

· Analyzer: Capillary gas chromatograph GC-2014 available from Shimadzu Corporation
· Analytical column: available from GL Sciences Inc., HP-5 (30 m, 0.32 mm I.D., film thickness 0.25 µm
· Oven temperature: 50°C (5 minutes) - temperature raising rate 20°C/min -280°C (14.5 minutes)
· Detector: FID, temperature 280°C

### <GC-MS measurement conditions>

· Analyzer: available from Agilent Technologies, GCMS-7890/5975
· Ionization voltage: 70eV
· Analytical column: available from Agilent Technologies, DB-5MS UI (30 m, 0.25 mmI.D., film thickness 0.25 µm)
· Oven temperature: 50°C (0 minutes) - temperature raising rate 5°C/min -150°C (0 minutes) - temperature raising rate 20°C/min -320°C (5 minutes)

### <1H-NMR spectrum analysis>

· Instrument: 1H-NMR spectrum instrument available from Bruker AVANCE NEO 500 MHz
· Internal standard substance: Tetramethylsilane (TMS)

### <Yield, Selectivity>

An area ratio (GC%) of a target product was determined by gas chromatography analysis, and the yield and selectivity of the target product were calculated by the following formula by an internal standard method.
· Yield (mol%) = Amount acquired (mol) of target product/Amount (mol) of ingredients charged × 100
· Selectivity (mol%) = Amount acquired (mol) of target product/Reaction amount (mol) of ingredients × 100

### <Synthesis Example 1>

### (Synthesis method of methyl-5-methylfuran-2-carboxylate [Furoate production step])

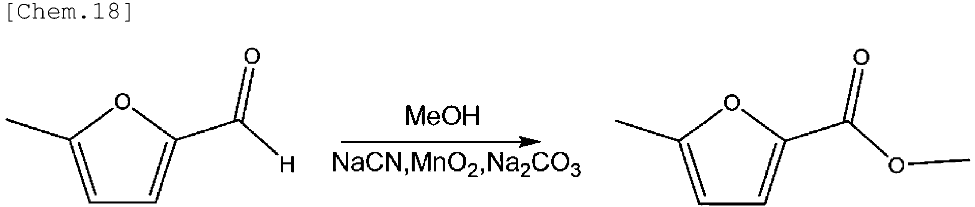

A 1000 mL three-necked flask equipped with a mechanical stirrer, a thermometer, and an exhaust gas line was charged with 66.0 g of 5-methylfurfural, 1.176 g of NaCN as a catalyst, and 214.0 g of methanol as a solvent, and the mixture was stirred at 25°C for 10 minutes at 300 rpm (revolutions per minute). After confirming that NaCN was dissolved, the reaction liquid was cooled, and 31.8 g of Na₂CO₃ and 188.0 g of MnO₂ were charged little by little over 20 minutes to 30 minutes while maintaining the temperature between 2 and 10°C. The temperature of the reaction liquid was raised to 40°C, and the reaction was performed at 300 rpm for 4 hours.

The reaction liquid was cooled, then subjected to solid-liquid separation, and rinsed five times with 40 mL of methanol, then 434.8 g of the resulting liquid was analyzed by gas chromatography by an internal standard method, and as a result, 80.7 g of the desired methyl-5-methylfuran-2-carboxylate was contained. The yield was 96.1 mol%, and the selectivity was 96.1 mol%.

When the produced liquid was rectified using a rectification column having a theoretical plate number of 5 (distillation temperature: 61°C, degree of vacuum: 0.4 kPa), a main fraction of 99.0GC% was produced in an amount of 75.9 g (distillation yield: 94.0 mol%) by gas chromatography analysis.

The produced fraction was analyzed by GC-MS, and as a result, the molecular weight of the target product was 140. In addition, ¹H-NMR chemical shift values (δ ppm, TMS reference) in a deuterated chloroform solvent were 2.38 (s, 3H), 3.87 (s, 3H), 6.12 (d, 1H), and 7.08 (d, 1H).

This identified the desired methyl-5-methylfuran-2-carboxylate.

### <Synthesis Example 2>

### (Synthesis method of methyl-5-methyltetrahydrofuran-2-carboxylate [Hydrogenated furoate production step])

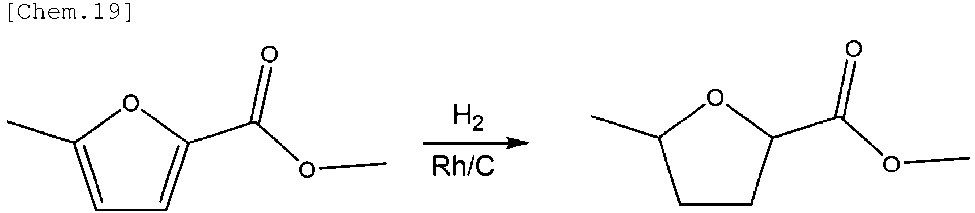

A hydrogenation reaction was performed using a stainless-steel autoclave having an internal volume of 75 mL and including a magnetic force induction type stirrer and three inlet nozzles at the top.

49.0 g of methyl-5-methylfuran-2-carboxylate prepared above and 3.0 g of a 5% Rh/C catalyst (water content: 50.5 mass%) were charged into an autoclave, and after replacement with nitrogen gas was performed three times, an airtightness test was performed at 6 MPa. After the pressure drop, the inside of the system was pressurized with H₂ gas, and the reaction was performed at 90°C and 4.5 MPa for 7.5 hours. After completion of the reaction, the resultant was subjected to solid-liquid separation, and rinsed three times with 10 mL of methanol, then 73.4 g of the resulting liquid was analyzed by gas chromatography by an internal standard method, and as a result, 45.4 g of the desired methyl-5-methyltetrahydrofuran-2-carboxylate was contained. The yield was 90.0 mol%, and the selectivity was 90.0 mol%.

When the produced liquid was rectified using a rectification column having a theoretical plate number of 5 (distillation temperature: 48°C, degree of vacuum: 0.2 kPa), a main fraction of 99.1GC% was produced in an amount of 41.2 g (distillation yield: 92.0 mol%) by gas chromatography analysis.

The produced fraction was analyzed by GC-MS, and as a result, the molecular weight of the target product was 144. In addition, the chemical shift value (δ ppm, TMS reference) of ¹H-NMR in the deuterated chloroform solvent was 1.34 (d, 3H), 1.53 (m, 1H), 2.01 (m, 1H), 2.10 (m, 1H), 2.23 (m, 1H), 3.74 (s, 3H), 4.19 (m, 1H), and 4.49 (m, 1H).

This identified the desired methyl-5-methyltetrahydrofuran-2-carboxylate.

### <Example 1>

### (Synthesis method of 1-hexyl-5-methyltetrahydrofuran-2-carboxylate [Transesterified hydrogenated furoate production step])

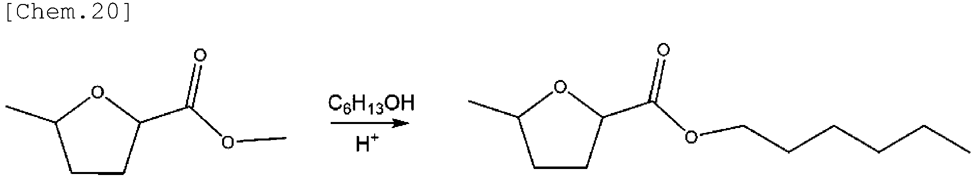

Into a 100 mL two-necked flask equipped with a Dean-Stark apparatus, a thermometer, and a stirrer, 20.1 g of methyl-5-methyltetrahydrofuran-2-carboxylate prepared above, 35.6 g of 1-hexanol (2.5 molar equivalent based on the substrate), and 0.80 g of p-toluenesulfonic acid (4 mass% based on the substrate) as a catalyst were charged, and the reaction was performed at 150°C for 4 hours at 500 rpm. Methanol produced as a by-product in the reaction was removed to the outside of the system during the reaction.

After completion of the reaction, water washing was performed once using 20 mL of a 0.2 mass% aqueous NaOH solution and twice using 20 mL of distilled water, then 49.9 g of the produced liquid was analyzed by gas chromatography by an internal standard method, and as a result, 27.5 g of the desired 1-hexyl-5-methyltetrahydrofuran-2-carboxylate was contained. The yield was 92.0 mol%, and the selectivity was 94.4 mol%.

When the produced liquid was rectified using a rectification column having a theoretical plate number of 5 (distillation temperature: 75°C, degree of vacuum: 0.13 kPa), a main fraction of 98.9GC% was produced in an amount of 25.0 g (distillation yield: 91.0 mol%) by gas chromatography analysis.

The produced fraction was analyzed by GC-MS, and as a result, the molecular weight of the target product was 214. In addition, the chemical shift value (δ ppm, TMS reference) of ¹H-NMR in the deuterated chloroform solvent was 0.88 (t, 3H), 1.30 (m, 6H), 1.34 (d, 3H), 1.60 (m, 3H), 2.16 (m, 3H), 4.25 (m, 3H), and 4.47 (q, 1H).

This identified 1-hexyl-5-methyltetrahydrofuran-2-carboxylate [compound represented by Formula (2-1)].

The produced fraction was different from a known flutate having only a fruity aromatic note or a known geranyl acetate having only a rose-like aroma, and was characterized in that the fraction was a novel aroma of an attractive jasmine floral having a herbal feel, and was superior in aroma persistence to other known esters or geranyl acetates.

### (Fragrance composition that gives natural feel and natural green note to white floral-like scent)

CIS-3-HEXENYL BENZOATE was compared as a benchmark with a Gardenia type formulation. In the formulation, 3 mass% of 1-hexyl-5-methyltetrahydrofuran-2-carboxylate was used (CIS-3-HEXENYL BENZOATE: 97 g, 1-hexyl-5-methyltetrahydrofuran-2-carboxylate: 3 g).

In the produced fragrance composition, in the scent evaluation by a perfumer, even in Gardenia, a very sharp green feel was strongly exhibited in a metallic manner, and a smooth natural feel was sharply exhibited. As time passed, a soft Jasmin tone having a texture of a white floral petal appeared. 1-Hexyl-5-methyltetrahydrofuran-2-carboxylate makes a slightly sweet and flat aromatic note of Gardenia more natural and deeper floral. It was confirmed that also in the lasting aromatic note, green having a natural feel improved palatability by matching with an aromatic note of Gardenia.

### <Example 2>

### (Synthesis method of 1-butyl-5-methyltetrahydrofuran-2-carboxylate [Transesterified hydrogenated furoate production step])

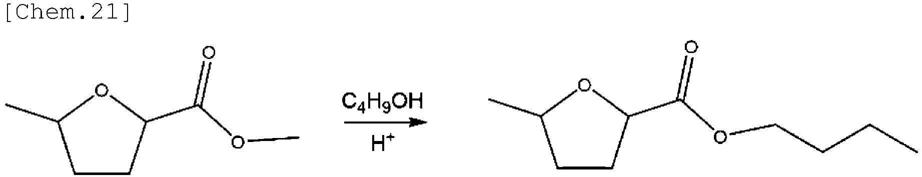

Synthesis and distillation were performed in the same manner as in Example 1 except that 1-butanol was used instead of 1-hexanol. The produced fraction was analyzed by GC-MS, so that the molecular weight of the target product was 186, and in addition, the chemical shift value (δ ppm, TMS reference) of ¹H-NMR in the deuterated chloroform solvent was 0.93 (t, 3H), 1.30 (d, 3H), 1.50 (m, 5H), 2.15 (m, 3H), 4.20 (m, 3H), and 4.47 (m, 1H). This identified the desired 1-butyl-5-methyltetrahydrofuran-2-carboxylate [compound represented by Formula (2)].

The produced fraction was different from a known flutate having only a fruity aromatic note or a known geranyl acetate having only a rose-like aroma, and was characterized in that the fraction was a novel aroma having a Jasmin-like floral and fruity component having a green feel.

### (Fragrance composition capable of imparting smooth floral feel and diffusibility with natural feel)

VELOUTONE was compared as a benchmark with a formulation of Osmanthus type. In the formulation, 2 mass% of 1-butyl-5-methyltetrahydrofuran-2-carboxylate was used (VELOUTONE: 98 g, 1-butyl-5-methyltetrahydrofuran-2-carboxylate: 2 g).

In the produced fragrance composition, in the scent evaluation by the perfumer, since an aromatic note of Osmanthus was characterized by Woody, the overall tone tended to sink slightly in VELOUTONE; however, 1-butyl-5-methyltetrahydrofuran-2-carboxylate shifted its Woody to a smooth floral feel and finished the scent with more spread. The lasting scent also imparted a body feel of Jasmin Floral, and it was confirmed that the composition was a material very effective for a floral-based aromatic note.

### <Example 3>

### (Synthesis method of 1-hexyl-5-methylfuran-2-carboxylate [Transesterified furoate production step])

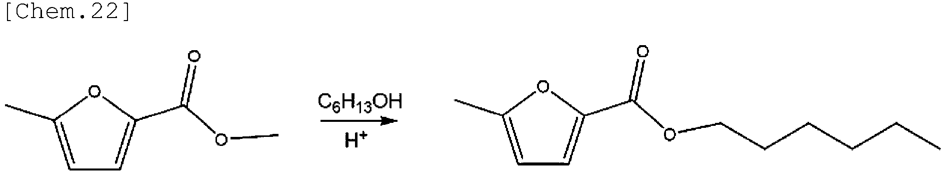

Synthesis and distillation were performed in the same manner as in Example 1 except that methyl-5-methylfuran-2-carboxylate was used instead of methyl-5-methyltetrahydrofuran-2-carboxylate. The produced fraction was analyzed by GC-MS, so that the molecular weight of the target product was 210, and in addition, the chemical shift value (δ ppm, TMS reference) of ¹H-NMR in the deuterated chloroform solvent was 0.89 (t, 3H), 1.36 (m, 6H), 1.72 (tt, 2H), 2.38 (s, 3H), 4.27 (t, 2H), 6.10 (d, 1H), and 7.06 (d, 1H). This identified the desired 1-hexyl-5-methylfuran-2-carboxylate [compound represented by Formula (1)].

The produced fraction was different from a known flutate having only a fruity aromatic note or a known geranyl acetate having only a rose-like aroma, and was characterized in that the fraction was a novel aroma like CIS-3-SALICYLATE that strongly gives a floral feel, and was superior in aroma persistence to other known esters or geranyl acetates.

### (Fragrance composition that gives natural feel to white floral-like scent and gives green note to lasting scent)

CIS-3-HEXENYL SALICYLATE was compared as a benchmark with a formulation of Hand & Body Lotion. In the formulation, 30 mass% of 1-hexyl-5-methylfuran-2-carboxylate was used (CIS-3-HEXENYL SALICYLATE: 70 g, 1-hexyl-5-methylfuran-2-carboxylate: 30 g).

The produced fragrance composition had an image in which soft and elegant floral-green was added as a whole in the scent evaluation by the perfumer. After one day, it was confirmed that the composition was the material exhibited a floral feel of a petal-like image having a natural feel unlike the effect of CIS-3-HEXENYL SALICYLATE.

### <Example 4>

### (Synthesis method of 2-ethylhexyl-5-methylfuran-2-carboxylate [Transesterified furoate production step])

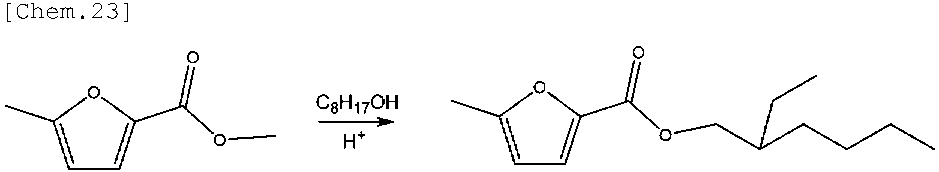

Synthesis and distillation were performed in the same manner as in Example 3 except that 2-ethylhexanol was used instead of 1-hexanol. The produced fraction was analyzed by GC-MS, so that the molecular weight of the target product was 238, and in addition, the chemical shift value (δ ppm, TMS reference) of ¹H-NMR in the deuterated chloroform solvent was 0.91 (m, 6H), 1.36 (m, 8H), 1.68 (ttt, 1H), 2.38 (s, 3H) 4.19 (dd, 2H), 6.10 (d, 1H), and 7.04 (d, 1H). This identified the desired 1-ethylhexyl-5-methylfuran-2-carboxylate [compound represented by Formula (1)].

The produced fraction was different from a known flutate having only a fruity aromatic note or a known geranyl acetate having only a rose-like aroma, was soft Green Floral, also had a Balsamic feel, was a novel aroma like BENZYL SALICYLATE having a very good quality, and had excellent aroma persistence as compared with other known esters or geranyl acetate.

### (Fragrance composition capable of imparting white floral feel and diffusibility with natural feel)

BENZYL SALICYLATE was compared as a benchmark with a Jasmin type formulation. In the formulation, 10 mass% of 1-ethylhexyl-5-methylfuran-2-carboxylate was used (BENZYL SALICYLATE: 90 g, 1-ethylhexyl-5-methylfuran-2-carboxylate: 10 g).

In the produced fragrance composition, in the scent evaluation by the perfumer, an elegant floral feel was added to an animalic portion and a cheap image, and a smooth natural feel was imparted. As time passed, a texture of a white petal of Jasmin is well represented. It was confirmed that the lasting scent was a material in which Fatty having a natural feel enhanced the texture by sumptuously producing a base note of Jasmin.

### <Comparative Example 1>

### (Synthesis method of ethyl-5-methylfuran-2-carboxylate)

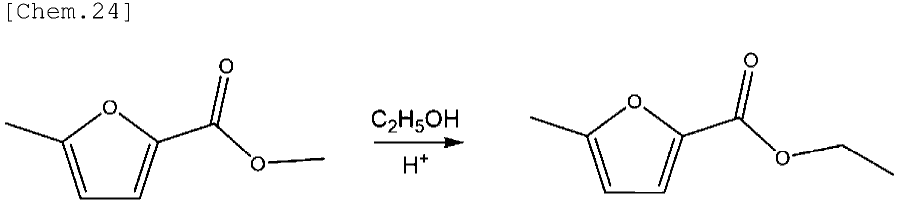

Synthesis and distillation were performed in the same manner as in Example 3 except that ethanol was used instead of 1-hexanol. As a result of analyzing the produced fraction by GC-MS, the molecular weight of the target product was 154, and 1-ethyl-5-methylfuran-2-carboxylate was identified.

The resulting fraction was a pungent and fatty green note. The lasting scent was weak, and the characteristics were not clear; therefore, it was determined that it was difficult to use the fraction as a fragrance.

### <Example 5>

### (Synthesis method of 2-ethylhexyl-5-methyltetrahydrofuran-2-carboxylate [transesterified furoate production step])

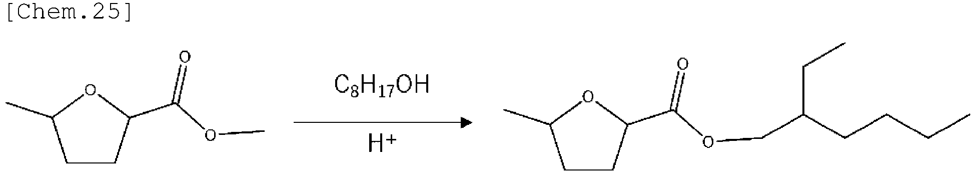

Synthesis and distillation were performed in the same manner as in Example 1 except that 2-ethylhexanol was used instead of 1-hexanol. The produced fraction was analyzed by GC-MS, so that the molecular weight of the target product was 242, and in addition, the chemical shift value (δ ppm, TMS reference) of ¹H-NMR in the deuterated chloroform solvent was 0.89 (m, 6H), 1.34 (m, 10 H), 1.59 (m, 3H), 2.13 (m, 3H) 4.07 (m, 3H), and 4.43 (q, 1H). This identified the desired 2-ethylhexyl-5-methyltetrahydrofuran-2-carboxylate [compound represented by Formula (3)].

### Industrial Applicability

Since the fragrance of the present embodiment contains a compound useful as a fragrance, the fragrance is useful as a fragrance component for a wide range of products.

## Claims

1. A fragrance, comprising a compound represented by Formula (1) and/or a compound represented by Formula (2): 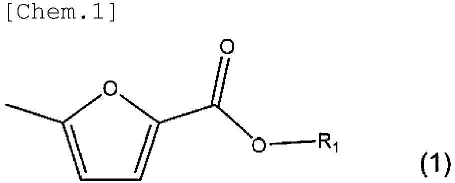 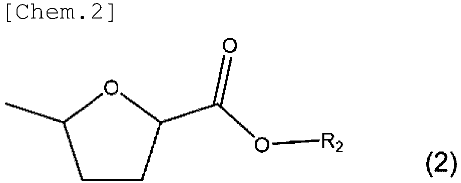 where in Formula (1) or Formula (2), R₁ and R₂ each independently represent a linear, branched, or cyclic alkyl group having from 3 to 12 carbon atoms, wherein the alkyl group optionally contains an unsaturated bond.

2. The fragrance according to claim 1, wherein in Formulas (1) and (2), R₁ and R₂ each independently represent a linear or branched alkyl group having from 3 to 12 carbon atoms.

3. The fragrance according to claim 1, wherein in Formula (1), R₁ is an n-hexyl group or a 2-ethylhexyl group.

4. The fragrance according to claim 1 or 2, wherein in Formula (2), R₂ is an n-butyl group or an n-hexyl group.

5. A fragrance composition, comprising the fragrance described in claim 1 or 2.

6. The fragrance composition according to claim 5, further comprising at least one fragrance component selected from the group consisting of: hydrocarbons having a terpene skeleton; alcohols; phenols; esters; aldehydes; ketones; acetals; ketals; ethers; nitriles; lactones; hydrocarbons; musks; natural fragrances; natural essential oils; natural extracts; plant extracts; and animal fragrances, with the proviso that compounds corresponding to the compound represented by Formula (1)/or the compound represented by Formula (2) are excluded.

7. A method for producing a fragrance, the method comprising:
a furoate production step of producing methyl-5-methylfuran-2-carboxylate by reacting 5-methylfurfural with methanol; and
a transesterified furoate production step of producing a fragrance containing a compound represented by Formula (1) set forth below by reacting the methyl-5-methylfuran-2-carboxylate with an alcohol: 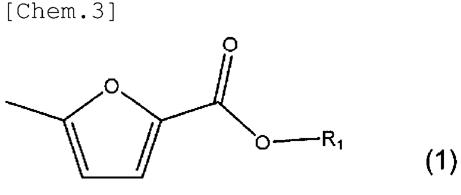
where in Formula (1), R₁ represents a linear, branched, or cyclic alkyl group having from 3 to 12 carbon atoms.

8. The method for producing a fragrance according to claim 7, wherein in Formula (1), R₁ represents a linear or branched alkyl group having from 3 to 12 carbon atoms.

9. A method for producing a fragrance, the method comprising:
a furoate production step of producing methyl-5-methylfuran-2-carboxylate by reacting 5-methylfurfural with methanol;
a hydrogenated furoate production step of producing methyl-5-methyltetrahydrofuran-2-carboxylate by reacting the methyl-5-methylfuran-2-carboxylate with H₂; and
a transesterified hydrogenated furoate production step of producing a fragrance containing a compound represented by Formula (2) set forth below by reacting the methyl-5-methyltetrahydrofuran-2-carboxylate with an alcohol: 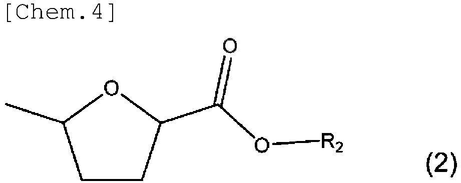
where in Formula (2), R₂ represents a linear, branched, or cyclic alkyl group having from 3 to 12 carbon atoms.

10. The method for producing a fragrance according to claim 9, wherein in Formula (2), R₂ represents a linear or branched alkyl group having from 3 to 12 carbon atoms.

11. A compound, represented by Formula (2-1) set forth below:

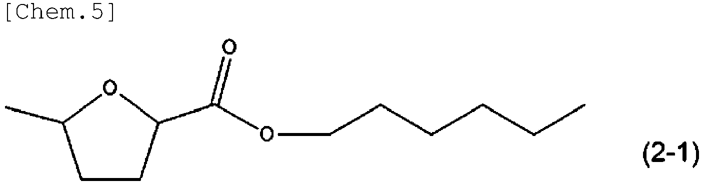

12. A compound, represented by Formula (2-2) set forth below:
